# EUROPEAN PATENT APPLICATION

(11) **EP 4 556 013 A1**
(43) Date of publication of application: **21.05.2025**
(21) Application number: 23839885.3
(22) Date of filing: 14.07.2023
(51) Int. Cl.: A61K 35/30, A61K 9/00, A61K 31/7088, A61P 27/02, A23L 33/10

(54) **PHARMACEUTICAL COMPOSITION FOR TREATMENT OF EYE DISEASES COMPRISING RETINAL ORGANOID-DERIVED EXOSOMES**

(30) Priority: 15.07.2022 KR 20220087475
(71) Applicant: Singularity Biotech Co., Ltd., Bucheon-si, Gyeonggi-do 14585 (KR)
(72) Inventor: PARK, Tae Kwann, Seoul 08089 (KR); CHANG, Hun Soo, Seoul 02714 (KR); HAN, Jung Woo, Seoul 05698 (KR); KIM, Yu Kyung, Seoul 08089 (KR)
(74) Representative: Crow, Martin
(86) International application number: PCT/KR2023/009680
(87) International publication number: WO 2024/014792

(57) **Abstract**

The present invention relates to a pharmaceutical composition and a food composition, for the prevention or treatment of degenerative retinal diseases, corneal epithelium wounds, and dry eye syndrome, comprising retinal organoid-derived exosomes.

## Description

### TECHNICAL FIELD

The present invention relates to a pharmaceutical composition, a pharmaceutical preparation and a food composition for the prevention or treatment of eye diseases including degenerative retinal diseases and ocular surface diseases, including exosomes derived from retinal organoids.

### BACKGROUND ART

The retina is a transparent nervous tissue that covers the innermost layer of the eye. Light entering the eye passes through the inner layer of the retina and is sensed by the retina's optic cells. Meanwhile, retinal nerve degenerative disease may occur due to congenital or acquired damage to the retina or complications of chronic diseases such as high blood pressure and diabetes. These diseases cause symptoms such as decreased vision, visual disturbances, night blindness, color blindness, color blindness and photopsia (light appears to flash when the eyes are moved).

Recently, Stargardt disease gene therapy targeting retinal pigment epithelial cells has been applied clinically, but it is limited to patients with some genetic mutations, and the current situation is that there is no technology to restore visual function by regenerating the degenerated retina.

The cornea is the outermost surface of the eye exposed to the external environment and is a transparent, blood-free tissue. Meanwhile, if trauma occurs in the cornea due to an external stimulus, corneal abrasion, keratitis or corneal opacity may occur. Current methods of treating corneal epithelial wounds include lubrication using artificial tears and eye ointments and supportive care such as bandage contact lenses. These treatments are often not effective for permanent wounds.

Recently, there has been research showing that exosomes derived from mesenchymal stem cells (MSCs) are effective in treating eye diseases. However, since the cornea and retina are ectoderm, whereas mesenchymal stem cells are connective tissues derived from mesoderm, it can be expected that exosomes derived from mesenchymal stem cells will have limited effects in treating eye diseases, and it can be expected that the derived exosomes will have improved therapeutic effects.

Under this background, the present invention has demonstrated that the prevention or treatment effect of eye diseases can be improved by injecting retinal organoid-derived exosomes into retina and corneal tissue.

### DISCLOSURE

### TECHNICAL PROBLEM

The inventors of the present invention successfully isolated and extracted exosomes from a culture medium of early retinal organoids and injected the same into the vitreous cavity of rats to see if they had a therapeutic effect on retinal diseases. A significant reduction in cell death and a photoreceptor protection effect were confirmed at 2 and 4 weeks after injection, respectively. In addition, the inventors of the present invention wanted to see if it had a therapeutic effect on corneal wounds and dry eye syndrome by instilling the same on the ocular surface of rats. Wound healing, epithelial cell proliferation and anti-inflammatory effects were confirmed 24 and 36 hours after injection.

These results suggest the possibility that exosomes derived from early retinal organoids can be used to prevent or treat eye diseases, including degenerative retinal diseases and ocular surface diseases.

Accordingly, an object of the present invention is to provide a pharmaceutical composition for preventing or treating an eye disease, including exosomes derived from retinal organoids.

Another object of the present invention is to provide a pharmaceutical preparation for preventing or treating an eye disease, including the pharmaceutical composition.

Still another object of the present invention is to provide a food composition for preventing or ameliorating an eye disease, including exosomes derived from retinal organoids.

### TECHNICAL SOLUTION

As used herein, the term "prevention" refers to all actions that suppress or delay the onset of corneal epithelial wounds by administering the pharmaceutical composition according to the present invention.

As used herein, the term "treatment" refers to all actions in which the symptoms of degenerative retinal disease and corneal disease are improved or beneficially changed by administration of the pharmaceutical composition according to the present invention.

As used herein, the term "amelioration" refers to any action in which the symptoms of a disease are ameliorated or completely cured by administration of the composition according to the present invention.

As used herein, the term "subject" refers to a subject in need of treatment for a disease, and more specifically refers to a human or non-human primate, a mammal such as a mouse, a rat, a dog, a cat, a horse, cattle and the like, but is not limited thereto.

As used therein, the term 'include' means that other components may be further included rather than excluding other components, unless specifically stated to the contrary.

Hereinafter, the present invention will be described in more detail.

The present invention relates to a pharmaceutical composition for preventing or treating an eye disease, including exosomes derived from retinal organoids.

The retinal organoids may be derived from human induced pluripotent stem cells (hiPSC) and embryonic stem cells (ESC), but are not limited thereto.

In the present invention, the retinal organoid-derived exosomes may include at least one miRNA selected from the group consisting of hsa-miR-122-5p, hsa-miR-3591-3p, hsa-miR-99a-5p, hsa-miR-148a-3p, hsa-let-7a-5p, hsa-let-7b-5p, hsa-let-7c-5p, hsa-let-7f-5p, hsa-miR-125a-5p, hsa-miR-100-5p, hsa-miR-4443, hsa-miR-26a-5p, hsa-miR-128-3p, hsa-miR-27b-3p, hsa-miR-126-3p, hsa-miR-10a-5p, hsa-miR-99b-5p, hsa-let-7e-5p, hsa-miR-21-5p, hsa-miR-30d-5p, hsa-let-7g-5p, hsa-let-7i-5p, hsa-miR-423-5p, hsa-miR-3184-3p, hsa-miR-125b-5p, hsa-miR-92a-3p, has-miR- 92b-3p, hsa-miR-1246, hsa-miR-30a-5p, hsa-miR-30a-3p, hsa-miR-320b, has-miR- 22-3p, hsa-miR-423-3p, hsa-miR-151a-3p, hsa-miR-24-3p, hsa-miR-3184-5p, hsa-miR-9-5p, hsa-miR-191-5p, hsa-miR-1290, hsa-miR-181a-5p, hsa-miR-30e-3p, hsa-miR-25-3p, hsa-miR-320a, hsa-miR-203b-5p and hsa-miR-375, and preferably, it may include hsa-miR-122-5p, hsa-miR-3591-3p, hsa-miR-99a-5p, hsa-miR-148a-3p, hsa-let-7a-5p, hsa-let-7b-5p, hsa-let-7c-5p, hsa-let-7f-5p, hsa-miR-125a-5p, hsa-miR-100-5p, hsa-miR-4443, hsa-miR-26a-5p, hsa-miR-128-3p, hsa-miR-27b-3p, hsa-miR-126-3p, hsa-miR-10a-5p, hsa-miR-99b-5p, hsa-let-7e-5p, hsa-miR-21-5p and hsa-miR-30d-5p, but is not limited thereto.

In the present invention, the eye disease may be a degenerative retinal disease.

The degenerative retinal disease may include a hereditary retinal disease, an acquired degenerative retinal disease, retinopathy of prematurity, and optic neuropathy, but is not limited thereto.

The hereditary retinal disease may include retinitis pigmentosa, X-linked juvenile retinoschisis, Leber congenital amaurosis, Stargardt disease, choroideremia and chorioretinal atrophy (gyrate-atrophy), and the acquired degenerative retinal disease may include wet age-related macular degeneration, dry age-related macular degeneration, diabetic retinopathy, hypertensive retinopathy, retinal detachment, macular hole, macular telangiectasia (MacTel), retinal degeneration, macular degeneration, retinal vein occlusion, retinal artery occlusion and glaucoma, but is not limited thereto.

In the present invention, the eye disease may be an ocular surface disease of corneal epithelial tissue, stroma and endothelial tissue.

The ocular surface disease may include hereditary damage, inflammatory damage, immunological damage, traumatic damage, dry eye syndrome, conjunctival lesions, conjunctivitis, keratoconjunctivitis, corneal epithelial tissue damage, corneal stroma damage, corneal endothelial tissue damage, dry eye syndrome and eye strain, but is not limited thereto.

The corneal epithelial tissue damage may include corneal abrasion, keratitis and corneal dystrophy, and the corneal stromal damage and corneal endothelial tissue damage may include corneal laceration due to trauma, opacity, inflammatory keratitis, non-inflammatory keratitis and corneal dystrophy, but are not limited thereto.

The pharmaceutical composition for preventing or treating an ocular surface disease of corneal epithelial tissue, stroma and endothelial tissue may have effects of improving proliferation of corneal epithelial cells, corneal edema, opacity, immunosuppression and anti-inflammatory effects, but is not limited thereto.

In the present invention, the pharmaceutical composition may be for injection or topical administration to the eye.

The pharmaceutical composition or pharmaceutical preparation of the present invention may be administered orally or parenterally (e.g., intravenously, subcutaneously, intraperitoneally or applied topically, including to the eye) according to the desired method, and while the method of administration is not limited, it can preferably be administered directly to the eye through intraocular injection, including intravitreal injection and subretinal injection, or in the form of eye drops.

The dosage may vary depending on the patient's condition and weight, degree of disease, drug form, administration route and time, but it may be appropriately selected by a person skilled in the art.

The pharmaceutical composition of the present invention is administered in a pharmaceutically effective amount.

In the present invention, "a pharmaceutically effective amount" means an amount sufficient to treat or diagnose a disease with a reasonable benefit/risk ratio that is applicable to medical treatment or diagnosis, and the effective dose level may be determined based on factors including the type and severity of the patient's disease, drug activity, drug sensitivity, time of administration, route of administration and excretion rate, duration of treatment, concurrent medications and other factors well known in the medical field.

In an embodiment of applying the composition of the present invention to humans, the composition of the present invention may be administered alone, but may be administered by being generally mixed with a pharmaceutical carrier selected in consideration of the administration method and standard pharmaceutical practice.

The pharmaceutical composition of the present invention may further include pharmaceutically suitable and physiologically acceptable additives such as carriers, excipients and diluents.

Examples of carriers, excipients and diluents that may be included in the pharmaceutical composition of the present invention include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil and hyaluronic acid.

The pharmaceutical composition may be any one formulation selected from the group consisting of topical skin preparations, injections, infusions, sprays, liquids, ointments, suspensions, syrups, emulsions, powders, granules, tablets, sustained-release preparations, eye drops, capsules, intraocular implants, intraocular injections such as intravitreal or subretinal injections, contact lens cleaners and contact lens lubricants, but is not limited thereto.

When formulating, commonly used diluents or excipients such as fillers, extenders, binders, wetting agents, disintegrants and surfactants may be used. Solid preparations for oral administration include tablets, pills, powders, granules, capsules and the like, and these solid preparations may be prepared by mixing the extract with at least one excipient, such as starch, calcium carbonate, sucrose or lactose, gelatin and the like. In addition to simple excipients, lubricants such as magnesium styrate talc are also used.

Preparations for oral administration include suspensions, oral solutions, emulsions, syrups, ointments and the like, and in addition to the commonly used simple diluents such as water and liquid paraffin, various excipients such as wetting agents, sweeteners, fragrances and preservatives may be included.

Preparations for parenteral administration include sterilized aqueous solutions, non-aqueous solutions, suspensions, emulsions, freeze-dried preparations, eye drops and the like. Eye drops may be used in liquid, cream, gel or ointment form. Non-aqueous solvents and suspensions include propylene glycol, polyethylene glycol, vegetable oil such as olive oil and injectable ester such as ethyl oleate.

The pharmaceutical composition according to the present invention may be administered to mammals, including humans, through various routes. The administration method may be administered in any route commonly used, for example, oral, cutaneous, intravenous, intramuscular, subcutaneous, ocular surface and intraocular (intravitreal, subretinal, etc.) administration, and preferably, it may be administered in the form of an intraocular injection or eye drops.

The appropriate dosage of the pharmaceutical composition of the present invention varies depending on factors such as preparation method, administration method, patient's age, body weight, gender, pathological condition, food, administration time, administration route, excretion rate and reaction sensitivity, and usually, a skilled physician may easily determine and prescribe an effective dosage for the desired treatment or prevention.

The pharmaceutical composition of the present invention may be administered as an individual therapeutic agent or in combination with other therapeutic agents, may be administered sequentially or simultaneously with conventional therapeutic agents, and may be administered singly or multiple times. Considering all of the above factors, it is important to administer an amount that can achieve maximum effect with the minimum amount without side effects, and this may be easily determined by a person skilled in the art.

The administered dose of the pharmaceutical composition of the present invention may vary depending on the patient's age, weight, gender, dosage form, health condition and disease degree, and may be administered by dividing into once or several times a day at regular time intervals according to the judgment of a doctor or pharmacist.

Specifically, the effective amount of the pharmaceutical composition of the present invention may vary depending on the patient's age, gender, condition, weight, absorption, inactivation rate and excretion rate of the active ingredient in the body, type of disease and concomitant drug, and when it is used systemically, as a general exosome formulation, 1 × 10³ to 1 × 10²⁰ may be administered per individual, and in the case of topical use such as intraocular injection or use in the form of eye drops, it may be administered within the range of 1 × 10³ to 1 × 10²⁰. However, since the dosage may increase or decrease depending on the route of administration, severity of disease, gender, weight, age and the like, the above dosage does not limit the scope of the present invention in any way.

The present invention also relates a pharmaceutical preparation for preventing or treating an eye disease including a degenerative retinal disease, an ocular surface disease of the epithelial tissue, stroma and endothelial tissue of the cornea, including the pharmaceutical composition.

The pharmaceutical preparation may be a gene therapy agent, but is not limited thereto.

The degenerative retinal disease may include hereditary retinal disease, acquired degenerative retinal disease, retinopathy of prematurity and optic neuropathy, but is not limited thereto.

The hereditary retinal disease includes retinitis pigmentosa, X-linked juvenile retinoschisis, Leber congenital amaurosis, Stargardt disease, choroideremia and chorioretinal atrophy (gyrate-atrophy), and the acquired degenerative retinal disease includes wet age-related macular degeneration, dry age-related macular degeneration, diabetic retinopathy, hypertensive retinopathy, retinal detachment, macular hole, macular telangiectasia (MacTel), retinal degeneration, macular degeneration, retinal vein occlusion, retinal artery occlusion and glaucoma, but is not limited thereto.

In the present invention, the eye disease may be an ocular surface disease of corneal epithelial tissue, stroma or endothelial tissue.

The ocular surface disease includes hereditary damage, inflammatory damage, immunological damage, traumatic damage, dry eye syndrome, conjunctival lesions, conjunctivitis, keratoconjunctivitis, corneal epithelial tissue damage, corneal stroma damage, corneal endothelial tissue damage, dry eye syndrome and eye strain, but is not limited thereto.

The corneal epithelial tissue damage includes corneal abrasion, keratitis and corneal dystrophy, and the corneal stroma damage and corneal endothelial tissue damage include corneal laceration due to trauma, opacity, inflammatory keratitis, non-inflammatory keratitis and corneal dystrophy, but are not limited thereto.

The present invention also relates to a food composition for preventing or improving an eye disease including a degenerative retinal disease, an ocular surface disease of epithelial tissue, stroma and endothelial tissue of the cornea, including exosomes derived from retinal organoids.

There are no special restrictions on the types of the food. Examples of food to which the substance can be added include meat, sausages, bread, chocolate, candies, snacks, confectionery, pizza, ramen, other noodles, gum, dairy products including ice cream, various soups, beverages, tea, drink, alcoholic beverages and vitamin complexes, and include all foods in the conventional sense.

The beverage may contain various flavors or natural carbohydrates as additional ingredients. The above-mentioned natural carbohydrates may include monosaccharides such as glucose and fructose, disaccharides such as maltose and sucrose, natural sweeteners such as dextrin and cyclodextrin, and synthetic sweeteners such as saccharin and aspartame. The ratio of the natural carbohydrates may be appropriately determined by the selection of a person skilled in the art.

In addition to the above, the food composition of the present invention may contain various nutrients, vitamins, electrolytes, flavors, colorants, pectic acid and salts thereof, alginic acid and salts thereof, organic acids, protective colloidal thickeners, pH adjusters, stabilizers, preservatives, glycerin, alcohol, carbonating agents used in carbonated drinks and the like. In addition, the food composition of the present invention may contain pulp for the production of natural fruit juice, fruit juice drinks and vegetable drinks. These ingredients may be used independently or in combination. The proportions of these additives may also be appropriately selected by those skilled in the art.

In the food composition of the present invention, content that overlaps with the pharmaceutical composition is omitted in consideration of the complexity of the present specification.

### ADVANTAGEOUS EFFECTS

The present invention relates to a pharmaceutical composition for preventing or treating an eye disease, including exosomes derived from retinal organoids, and the composition of the present invention can be used as a treatment that is capable of suppressing photoreceptor loss in degenerative retinal diseases and suppressing the proliferation and inflammation of corneal epithelial cells.

### DESCRIPTION OF DRAWINGS

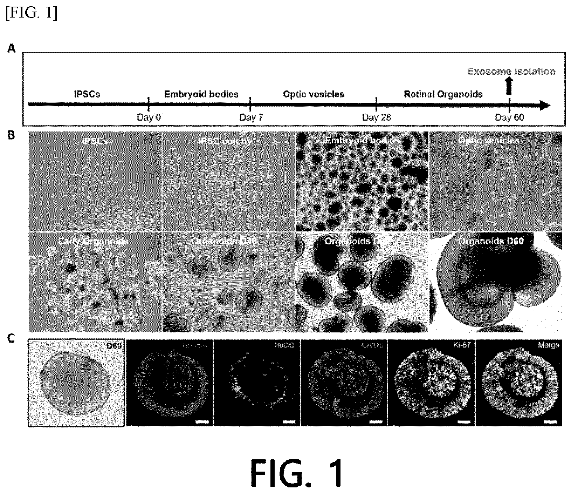

FIG. 1 shows a method for producing retinal organoids using human induced pluripotent stem cells according to an embodiment of the present invention (A), the shape of retinal organoids by culture period (B), and immunofluorescence staining results for the internal structure and retinal cell markers of 60-day cultured retinal organoids (C).

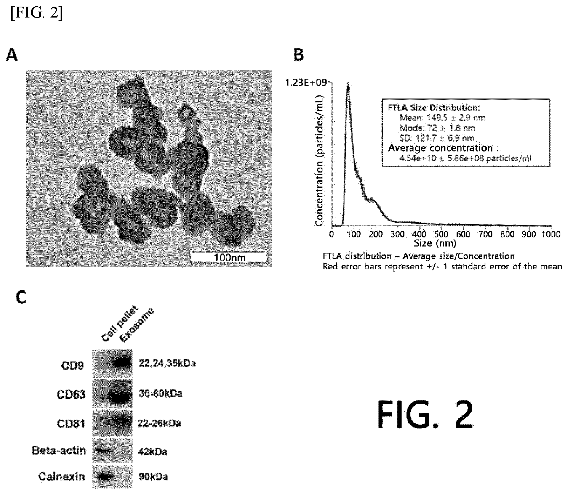

FIG. 2A shows the results of electron microscopy of exosomes isolated from the differentiated retinal organoid culture supernatant according to an embodiment of the present invention. As a result of electron microscopy, a typical cup-shaped exosome shape was observed.
FIG. 2B shows the size distribution and concentration of exosomes isolated from the differentiated retinal organoid culture supernatant according to an embodiment of the present invention. The size distribution was confirmed to be homogeneous, with an average of 149.5 +/- 29 nm and a mode of 72 +/- 1.8 nm.
FIG. 2C shows Western blot analysis of exosomal markers CD63, CD81, and CD9 and cytoplasmic markers beta-actin and calnexin in exosomes of hiPSC-derived retinal organoids.

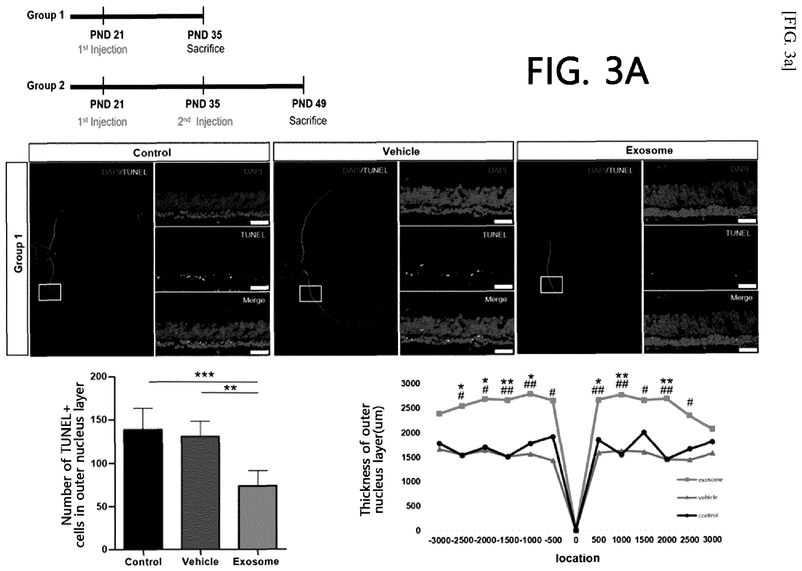

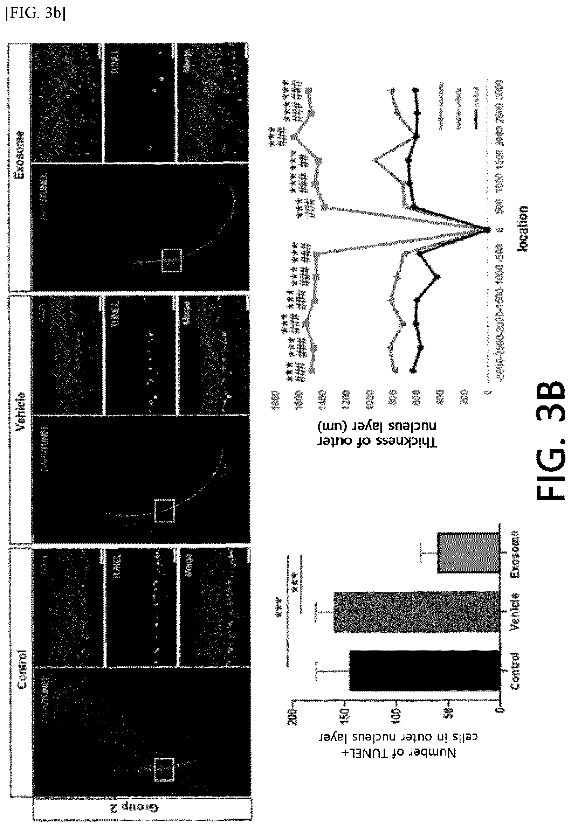

FIGS. 3a and 3b show the definition of an experimental group using Royal College of Surgeons (RCS) rats, the results of immunofluorescence staining (terminal deoxynucleotidyl transferase dUTP nick end labeling; TUNEL) in a control group, a vehicle (PBS, phosphate buffered saline)-treated group and a retinal organoid-derived exosome-treated group in the eye and the control group for retinal cell death, and the measurement of the thickness of the outer neuroblastic layer (ONL) of the retina. H in FIG. 3c shows the quantitative analysis of the amplitude changes of a- and b-waves according to various light intensity stimuli, and I in FIG 3c shows the visual acuity measured through optomotor response tests of the retina treated with the control group, the vehicle and retinal organoid-derived exosomes (Exo-RO) (n = 6 per group, *p < 0.05, **p < 0.01, two-way ANOVA).

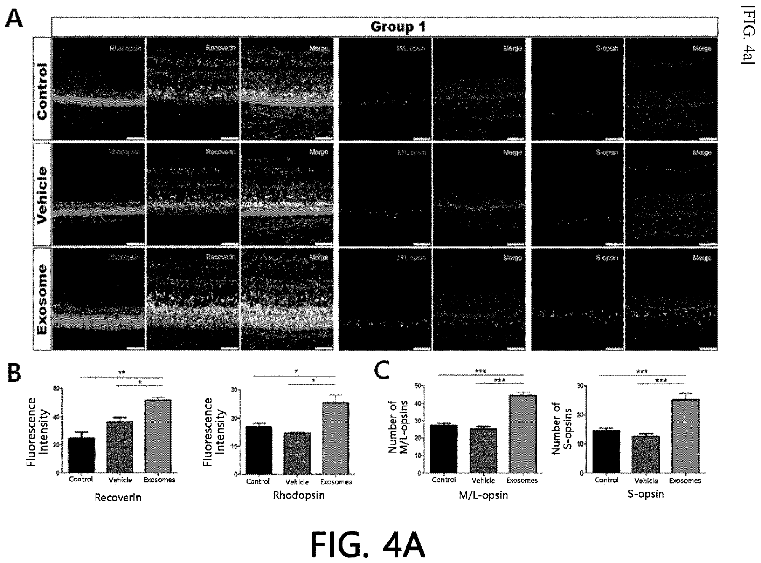

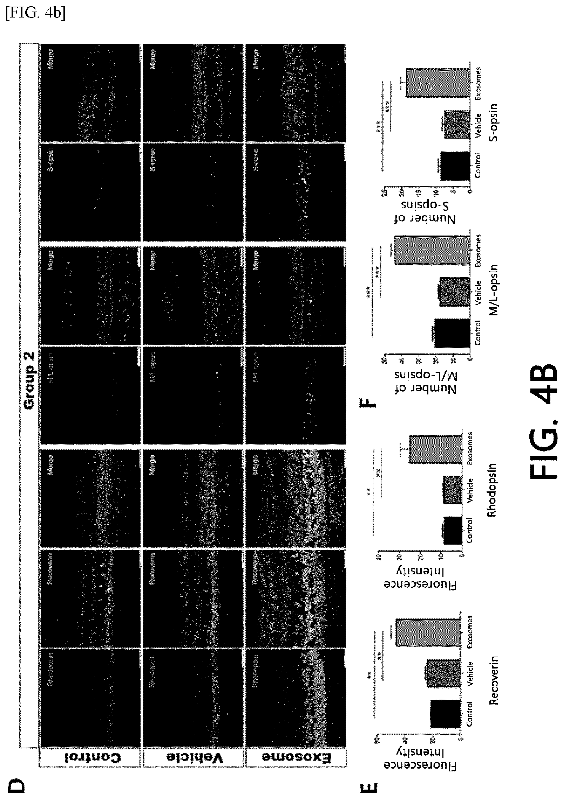

FIGS. 4a and 4b show the results of immunofluorescence staining of recoverin, rhodopsin (M/L-opsin) and S-opsin, which are markers of photoreceptor cells, in the retinas of the control group, vehicle-treated group and retinal organoid-derived exosome-treated group of RCS rats according to one embodiment of the present invention. A, B and C in FIG. 4a are the results of Experimental Group 1, and D, E and F in FIG. 4b are the results of Experimental Group 2.

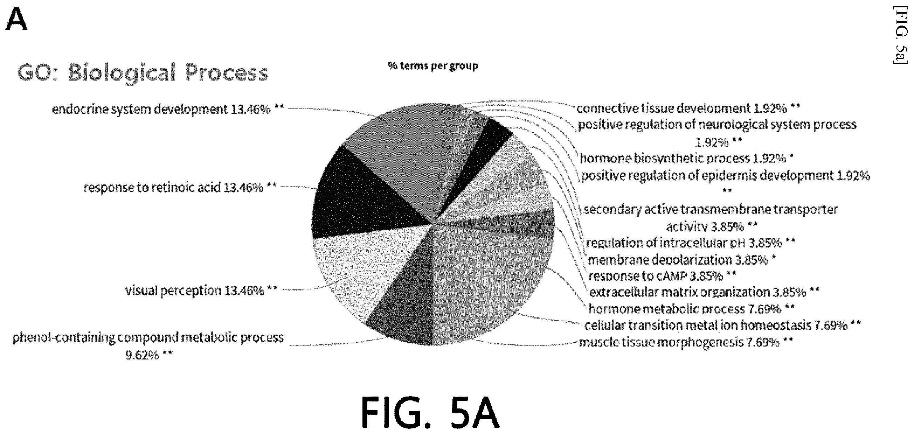

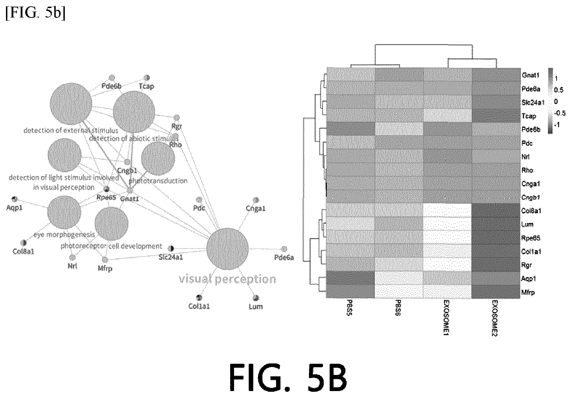

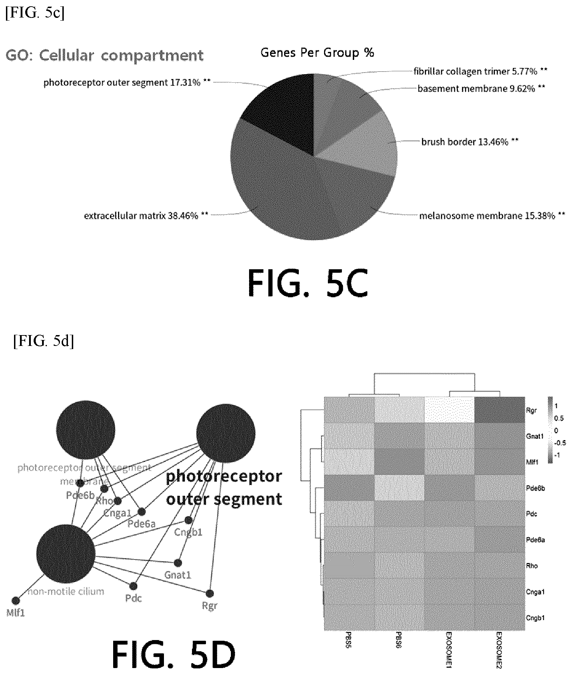

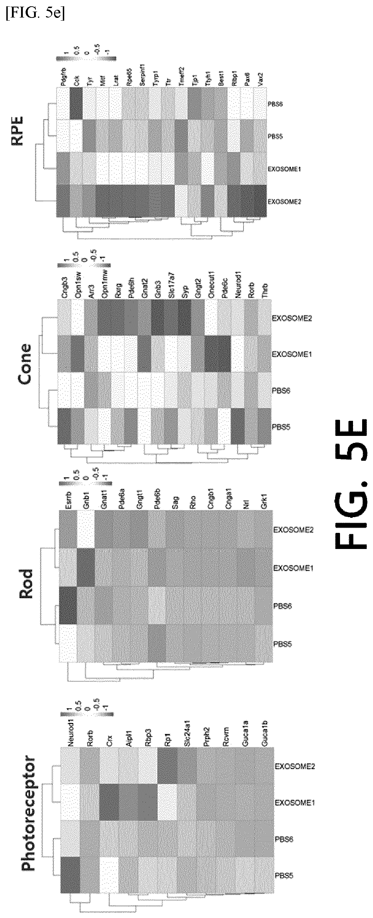

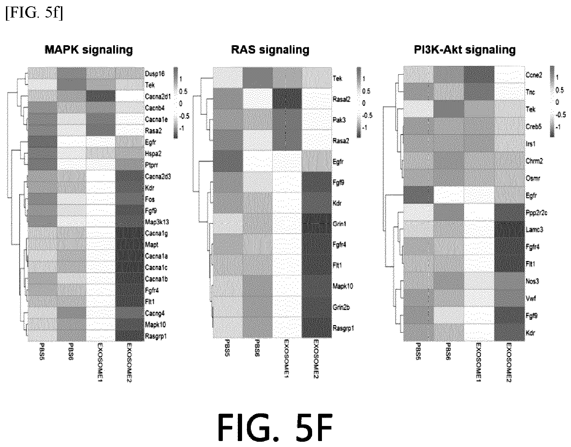

FIG. 5a is a biological pathway gene ontology of genes whose expression was increased among the results of total RNA sequence analysis of gene expression in RCS rat retina by treatment with exosomes derived from retinal organoids. FIG. 5b is a comparison of the core gene network and expression level of biological pathway gene ontology of genes increased in the exosome treatment group. FIG. 5c is a gene ontology for the intracellular action sites of genes increased in the exosome treatment group. FIG. 5d is a comparison of the core gene network and expression level of gene ontology for the intracellular action sites of genes increased in the exosome treatment group. FIG. 5e shows transcriptional profiling of retinal cell markers, and FIG. 5f shows that the expression of inflammation-related pathways, including mitogen-activated protein kinase (MAPK), phosphoinositide 3-kinase-Akt and RAS signaling, was significantly downregulated in retinas treated with retinal organoid-derived exosomes (logFC < -1, false discovery rate [q-value] < 0.05).

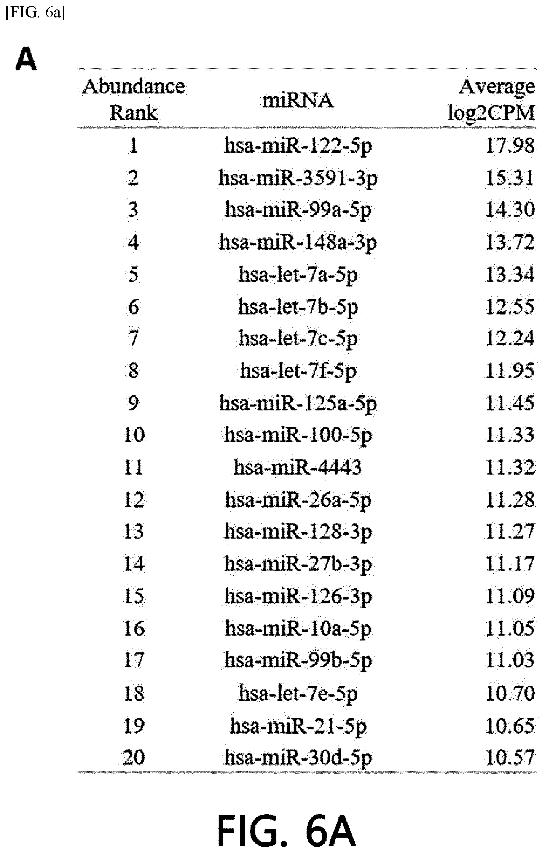

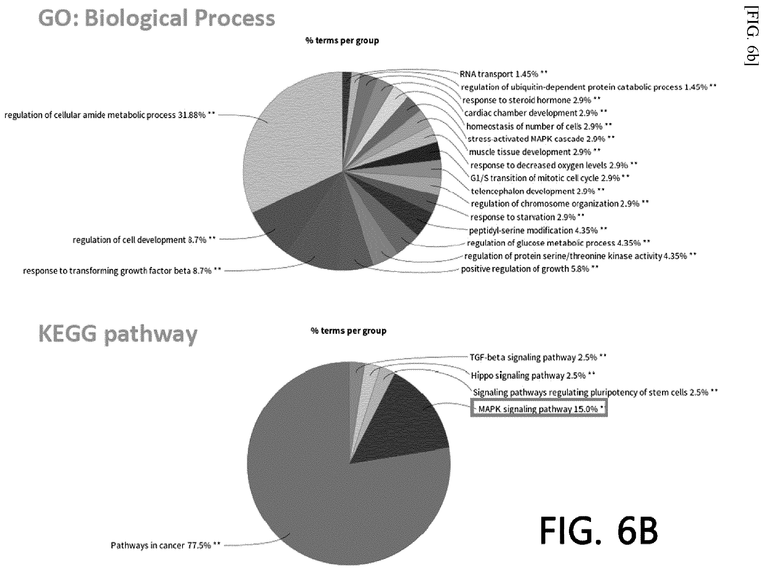

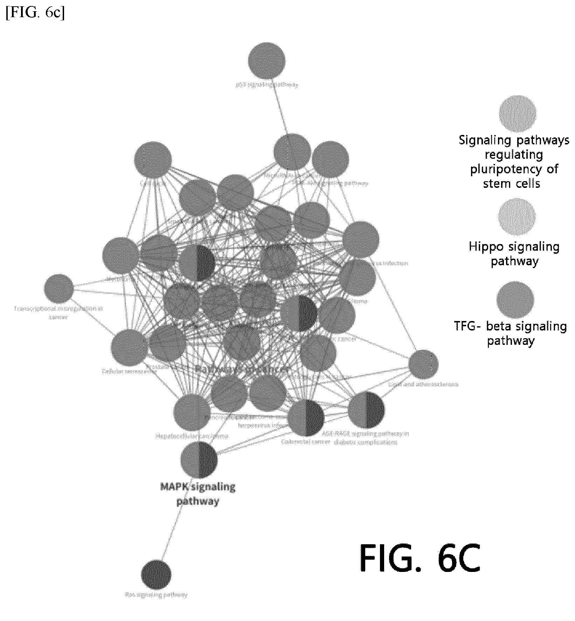

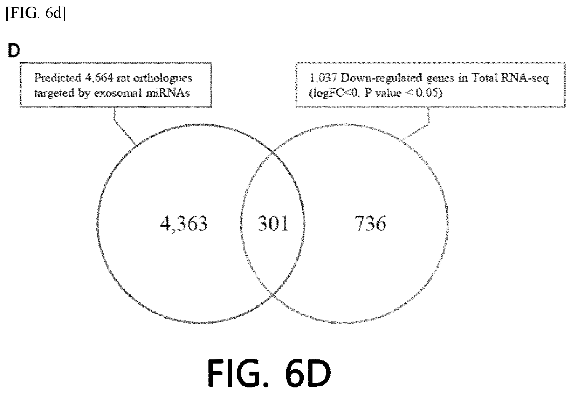

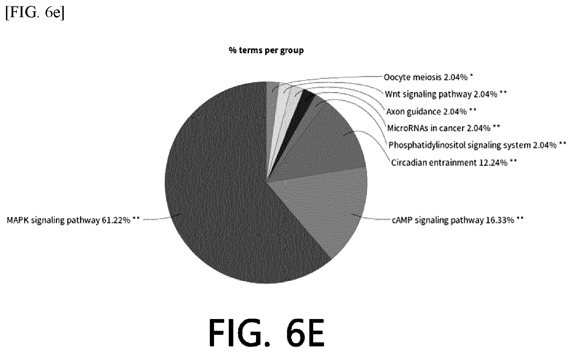

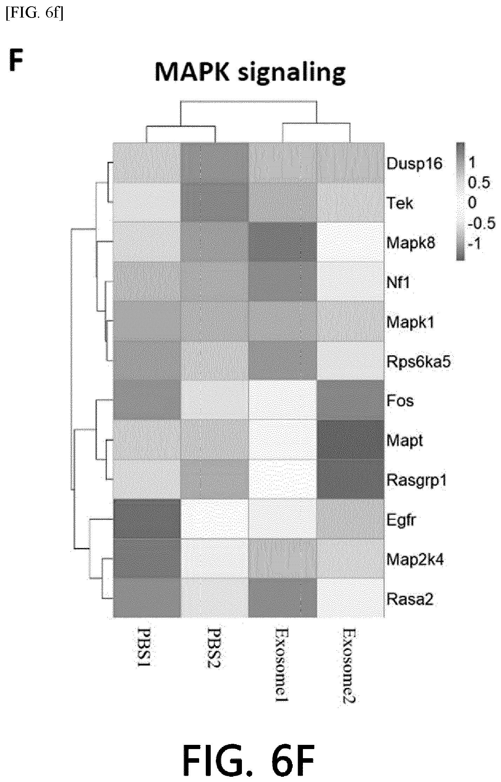

FIG. 6a shows the 20 most abundant miRNAs at more than 1,000 per million (cpm) in retinal organoid-derived exosomes, FIG. 6b shows the gene ontology and pathway enrichment graph of 293 genes predicted as shared targets of 10 or more of the 20 most abundant miRNAs, and FIG. 6c shows the network between molecular pathways involved in the target genes of exosomal miRNA derived from retinal organoids. FIG. 6d shows the results of extracting actual target genes by comparing predicted target genes and differentially expressed genes in Royal College of Surgeons (RCS) rats treated with exosomes derived from retinal organoids, and among 4,929 human target genes, 4,674 mouse homologous genes were identified, of which 301 genes were significantly downregulated in eyes treated with retinal organoid-derived exosomes compared to vesicle-treated eyes (p <0.05, logFC <0). FIG. 6e shows Kyoto Encyclopedia of Genes and Genomes (KEGG) pathway analysis, showing that 301 genes were mainly involved in the MAPK signaling pathway (61.22%). FIG. 6F shows significantly downregulated genes of the MAPK pathway targeted by the top 20 abundant miRNAs (cpm > 1,000) in retinal organoid-derived exosomes in the retina of RCS rats treated with retinal organoid-derived exosomes. (false discovery rate [q-value] <0.05).

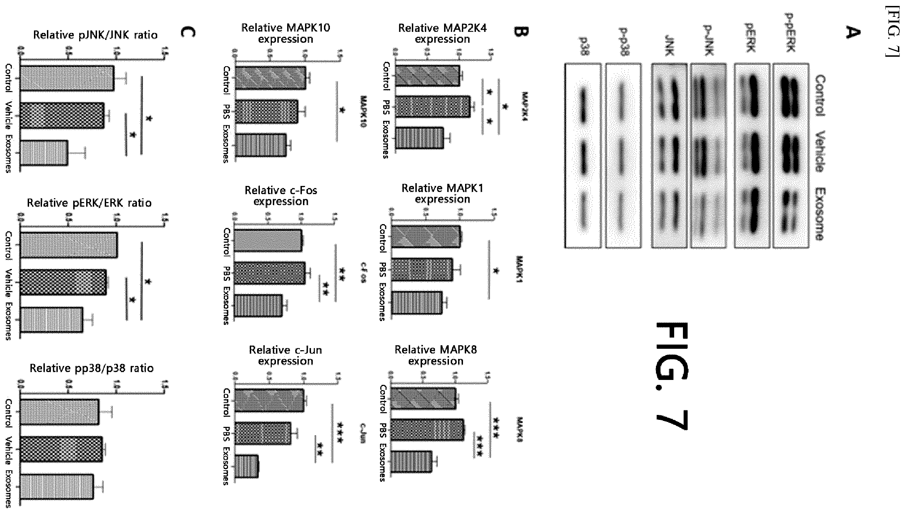

FIG. 7A is a quantitative analysis according to band density, and data are expressed as mean ± standard error (n = 3). FIG. 7B shows qRT-PCR results for the expression changes of MAPK pathway-related genes according to treatment groups in RCS rat retina. indicates. FIG. 7C shows the expression of the phosphorylated MAPK family assessed through Western blotting after treatment with exosomes derived from retinal organoids. The expressions of phosphorylated c-Jun N-terminal kinase (p-JNK)/JNK and phosphorylated extracellular signal-regulated kinase (pERK)/ERK were significantly decreased in retinas treated with retinal organoid-derived exosomes compared to the control and vehicle (PBS)-treated retinas (*p < 0.05, **p < 0.01, ***p < 0.001, one-way analysis of variance). FIG. 7D shows the effect of suppressing MAPK pathway activation by treating retinal organoid-derived exosomes in a retina pigment epithelium (RPE) cell model treated with H₂O₂ and causing oxidative damage.

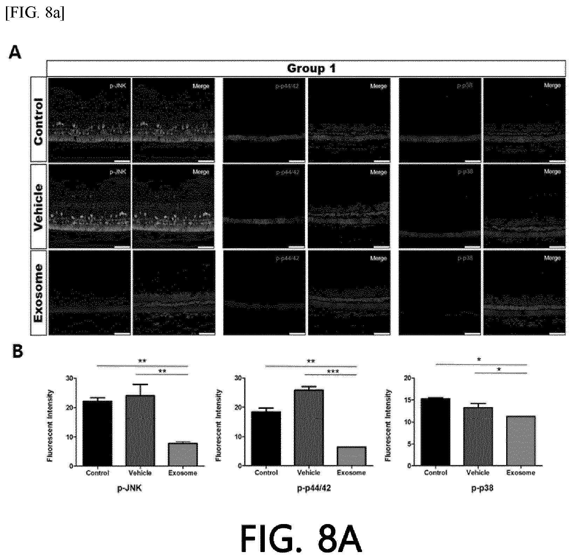

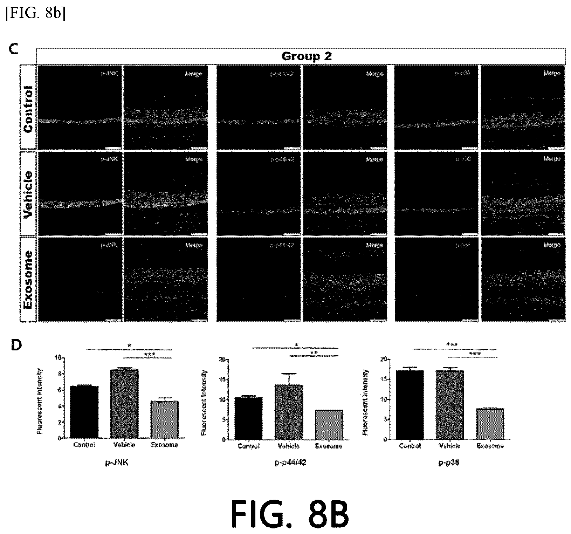

FIGS. 8a and 8b show immunofluorescence staining results and quantitative results for the effect of retinal organoid-derived exosomes on MAPK pathway activation in RCS rat retina according to an embodiment of the present invention. A and B in FIG. 8a are the results of Experimental Group 1, and C and D in FIG. 8b are the results of Experimental Group 2. In both of Groups 1 and 2, significant decreases in the expressions of p38, JNK, and p42/44 were observed in the intraocular administration group of retinal organoid-derived exosomes compared to the negative control and phosphate buffered solution (PBS) administration groups, indicating an overall inhibition of the expression of the MAPK pathway.

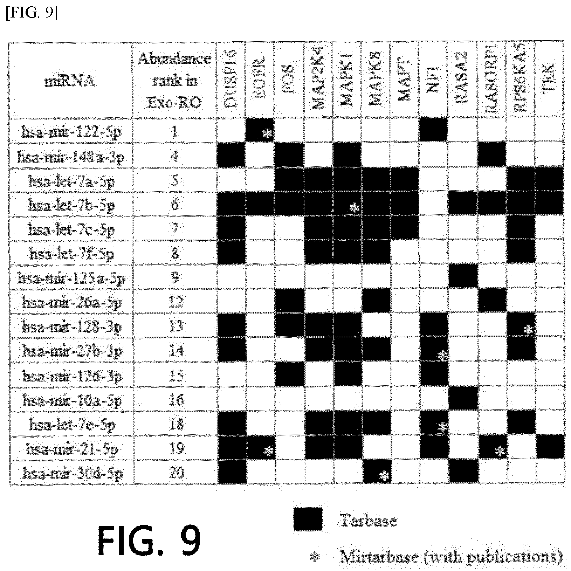

FIG. 9 shows miRNAs targeting 12 genes included in the gene ontology term "mitogen-activated protein kinase pathway" among the 20 miRNAs abundant in exosomes of retinal organoids.

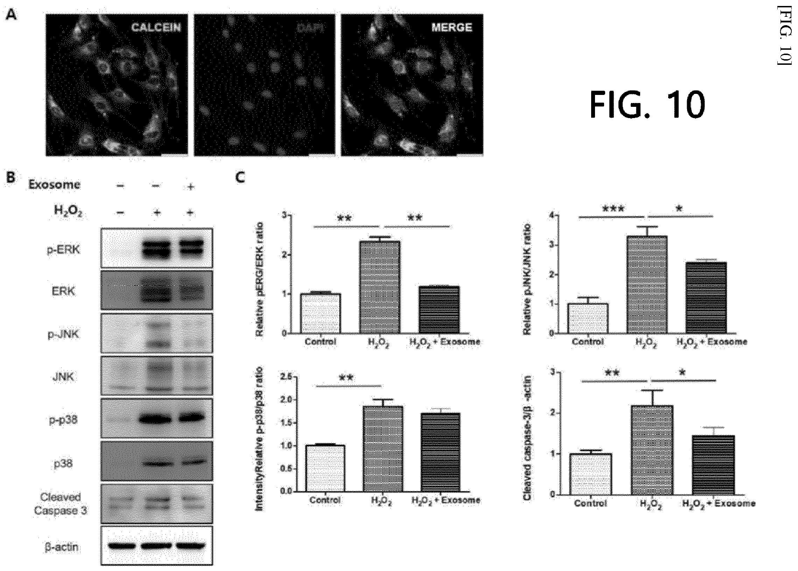

FIG. 10 shows an experiment on the effect of retinal organoid-derived exosomes on primary rat retinal pigment epithelium (RPE) cells after H₂O₂-mediated oxidative stress damage. FIG. 10A shows the uptake of retinal organoid-derived exosomes by RPE cells, and green fluorescein particles were observed throughout the cytoplasm. FIG. 10B is a quantitative analysis of band density, with data expressed as mean ± standard error (n = 3). FIG. 10C shows the Western blot analysis of phosphorylated mitogen-activated protein kinase (MAPK) and cleaved caspase-3 in H₂O₂-treated RPE cells treated with retinal organoid-derived exosomes, and it was confirmed that the treatment of retinal organoid-derived exosomes inhibited the expressions of phosphorylated c-Jun N-terminal kinase (p-JNK)/JNK, p-44/42 and cleaved caspase-3, compared to H₂O₂-treated RPE cells (*p < 0.05, **p < 0.01, ***p < 0.001, results by one-way analysis of variance).

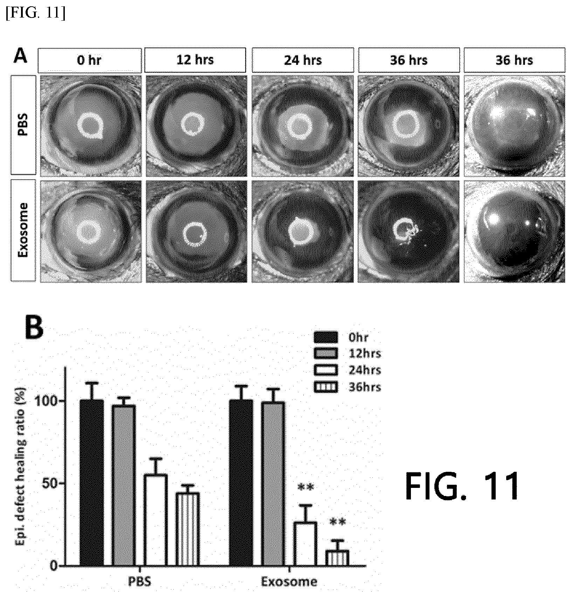

FIG. 11 shows the tissue photograph results of an experimental group treated with exosomes isolated from retinal organoids in a rat model with damaged epithelium according to an embodiment of the present invention, and a control group treated with a vehicle (PBS, phosphate buffer solution). FIG. 11A shows photographs 12, 24 and 36 hours after the treatment of the experimental group treated with retinal organoid-derived exosomes and the control group treated with a vehicle (PBS) after creating a 2 mm-sized defect in the corneal epithelium of a rat. FIG. 11B shows the wound healing rate (%) of the damaged cornea.

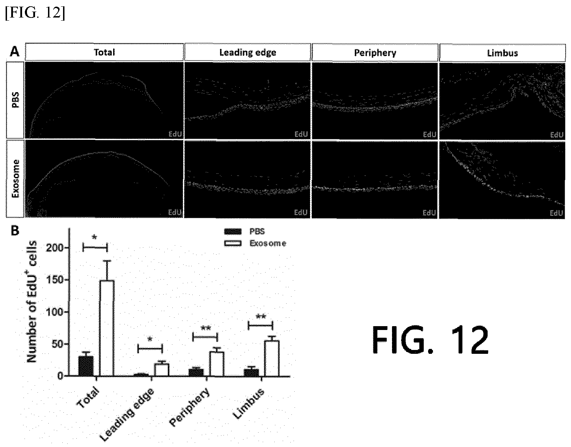

FIG. 12 shows the results of performing EdU irradiation to compare the degree of cell proliferation in the experimental group in which the cornea was treated with retinal organoid-derived exosomes and the control group in which the cornea was treated with a vehicle (PBS). FIG. 12A shows the degree of epithelial cell proliferation at the leading edge, periphery and limbus of the cornea. FIG. 12B shows a graph comparing the number of EdU⁺ cells.

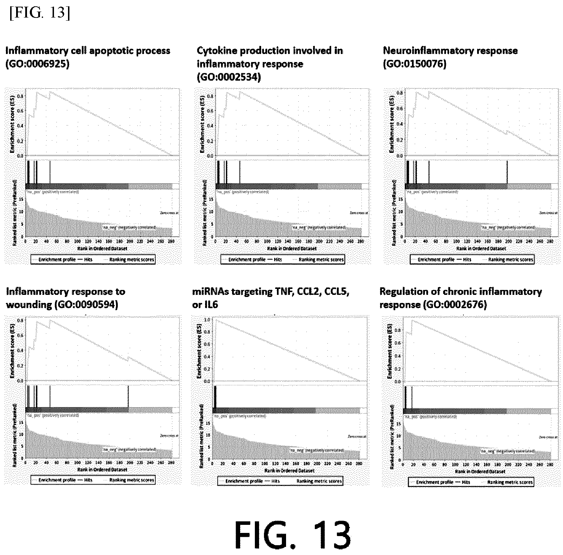

FIG. 13 shows the results of gene set enrichment analysis (GSEA) evaluating the anti-inflammatory potential of exosomal miRNA.

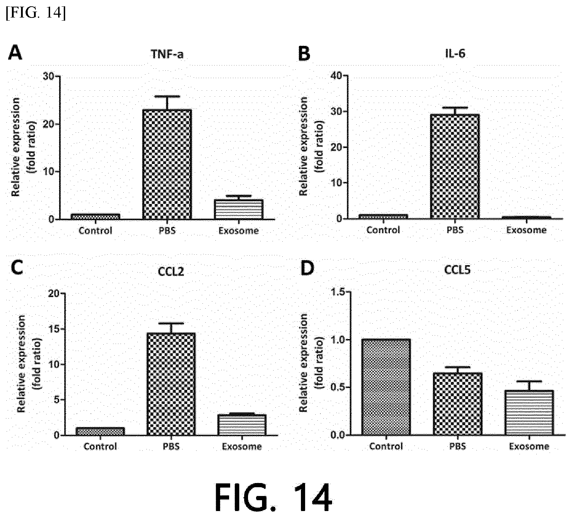

FIG. 14 shows the results of quantitative analysis comparing the expression levels of inflammatory cytokines, TNF-α, IL-6, CCL2 and CCL5, in the group treated with retinal organoid-derived exosomes to the cornea and the group treated with a vehicle using qRT-PCR.

### MODES OF THE INVENTION

Hereinafter, the present invention will be described in more detail through the following examples. However, these examples are only for illustrating the present invention, and the scope of the present invention is not limited by these examples.

### Experimental Example 1. Human induced pluripotent stem cell cultures

American Type Culture Collection (ATCC)-DYR0100 hiPSCs (ACS-1011, ATCC, Manassas, VA) were used. hiPSCs were maintained on vitronectin (Gibco CTS vitronectin; Thermo Fisher Scientific, Waltham, MA)-coated dishes with Essential 8 medium (Thermo Fisher Scientific). Cells were routinely cultured at 37°C in a standard 5% CO₂/95% air incubator with daily media replacement. Upon reaching approximately 70 to 80% confluency, cells were mechanically passaged with EDTA every 5 to 7 days. Isolated cell aggregates were collected in Essential 8 medium and carefully pipetted up and down to obtain a homogeneous suspension of cell aggregates that regenerated at a rate of 1/10 to 1/60 depending on confluence.

### Experimental Example 2. Differentiation into three dimensional ROs and collection of culture fluid

hiPSCs were maintained in 6-well vitronectin plates (Thermo Fisher Scientific). E8 medium (Thermo Fisher Scientific) was successfully used to maintain the health and pluripotency of all of hiPSCs.

In order to induce embryoid body (EB) formation on day 0, hiPSCs were separated by ReLeSR (STEMCELL Technologies, Vancouver, Canada) treatment, dissociated into single cells, and cultured in low attachment dishes in a 3:1 mixture of neural induction medium (NIM) including ROCK inhibitor Y27632 and DMEM/F12 (1:1, Thermo Fisher Scientific), 1% N-2 supplement, 1 X NEAA (non-essential amino acids) and 2 µg/mL heparin (Gibco). EB was gradually replaced by NIM in E8 medium.

On day 7, EBs were seeded into 6-well plates containing NIM with 10% FBS at a density of approximately 30 to 40 EBs/well. On day 16, the medium was changed from NIM to retinal differentiation medium (RDM) including DMEM/F12 (3:1, Welgene), 2% B27 supplement without vitamin A (Thermo Fisher Scientific), 1 x NEAA and penicillin/streptomycin (Thermo Fisher Scientific). On days 18 to 25, the loosely attached central portion of the nerve cluster was lifted and separated by pipetting with a P1000 pipettor. The collected aggregates were cultured in suspension with RDM, which can form three-dimensional optical cup structures. For long-term suspension cultures, the medium was supplemented with exosome-depleted fetal bovine serum (Thermo Fisher Scientific), 100 mM taurine (Sigma Aldrich, St. Louis, MO) and 2mM GlutaMAX (Thermo Fisher Scientific) on day 35. The cell media were changed every 2 to 3 days until the desired stage was reached.

Five organoids per plate were cultured in 1 mL of culture medium at a ratio of 1 organoid per 200 µL. On day 60, 500 µL each was collected from two culture solutions, and 1 mL of culture solution was collected to extract exosomes.

Afterwards, the collected medium was then centrifuged at 3,000 X g for 10 minutes to remove cell debris and stored frozen at -80°C until further analysis.

### Experimental Example 3. Exosome isolation

Exosomes were isolated from the culture supernatant of cultured organoids using the miRCURY exosome isolation kit (Qiagen, Hilden, Germany). The culture supernatant was collected from the organoid culture vessel and centrifuged at 3,000 xg for 10 minutes to remove cells and cell debris from the collected culture supernatant. After centrifugation, the supernatant was filtered through a 0.2 micrometer syringe filter, and 400 µL of buffer A per 1 mL of sample was added to the filtered supernatant. It was stirred to mix thoroughly, reacted at 4°C for 24 hours, and then centrifuged at 10,000 xg for 30 minutes at 20°C. After centrifugation, the supernatant was discarded, and 100 µL of buffer B was added to the pellet to suspend the same while stirring for 15 seconds. The suspension containing exosomes was stored at 4°C.

### Experimental Example 4. Animal preparation

All animal experiments were designed and performed in accordance with the Guide for the Care and Use of Laboratory Animals at the Association for Vision Research, Ophthalmic Statement on the Use of Animals in Ophthalmic and Vision Research, and were approved by the Animal Care and Use Committee of Soonchunhyang University Bucheon Hospital.

RCS/Kyo rats were used in the present invention and were purchased from Kyoto University. All rats were housed in pathogen-free rooms under standardized conditions and maintained in cages with free access to food and water in rooms with a 12/12 h light/dark cycle, with humidity and temperature maintained at 50% and 23 to 26°C, respectively. Anesthesia was administered by intraperitoneal injection of a 4:1 mixture of zoletyl and lumpun, and a 0.5% mixture of tropicamide and phenylephrine was instilled for mydriasis.

### Experimental Example 5. Nanoparticle tracking analysis

The particle size distribution of isolated exosomes was analyzed using a NanoSight NS300 system (Malvern Technologies, UK) consisting of a 488 nm laser and a high-sensitivity scientific CMOS camera. Samples were diluted 125 times in particle-free PBS (Gibco, Waltham, MA, USA) according to the manufacturer's recommendations and then analyzed. Data were analyzed using NTA 2.3 software with a detection threshold of 3.

### Experimental Example 6. Transmission electron microscopy (TEM)

Concentrated exosomes were added to a Formvar-coated copper grid (200 mesh grid formvar/carbon film) for 2 minutes and then fixed (negative stain) with 2% paraformaldehyde and 1% uranyl acetate. Exosomes were visualized using a transmission electron microscope (JEM-1400 FLASH) operating at 100 kV.

### Experimental Example 7. Western blot experiment to identify exosomal protein markers

Western blot was used to identify exosomal protein markers. Exosomal proteins (5 µg/well) were loaded on a 10% polyacrylamide gel and subjected to electrophoresis, and then transferred to a nitrocellulose membrane at 80 V for 120 minutes. The membrane was blocked with 5% skim milk for 1 hour at room temperature and washed with a solution containing 0.1% Tween 20 in TBS. Afterwards, it was incubated overnight at 4°C with the anti-CD9 monoclonal antibody 1:500 dilution solution (Cell Signaling Technology, Danvers, MA, USA), anti-CD63 monoclonal antibody 1:1,000 dilution solution (Santa Cruz Biotechnology, Dallas, TX, USA), anti- CD81 monoclonal antibody 1:500 dilution solution (Santa Cruz Biotechnology), anti-Calnexin monoclonal antibody 1:500 dilution solution (Cell Signaling Technology), and anti-β-actin monoclonal antibody 1:10,000 dilution solution (Sigma-Aldrich). The membrane was then incubated with a 1:5,000 dilution solution of horseradish peroxidase-conjugated secondary antibody (GenDEPOT, Baker, TX, USA) for 1 hour at room temperature. Proteins of interest were detected with enhanced chemiluminescence solution (Amersham Pharmacia Biotech, Buckinghamshire, UK) and visualized using the Azure Biosystems C280 digital system (Azure biosystems, Dublin, CA, USA).

### Experimental Example 8. Intravitreal injection

RCS rats were injected with retinal organoid-derived exosomes twice on days 21 and 35. Under surgical microscope observation, a 30G needle was used to make a hole approximately 2 mm behind the limbus. 2 µL of exosomes were injected using a NanoFil syringe with a 34-G beveled needle.

### Experimental Example 9. In vivo study design and treatment schedule

An *in vivo* study using RCS rats was designed with two different injection schedules. In Group 1, retinal organoid-derived exosomes or 0.1% PBS were intravitreally injected into one eye of RCS rats at postnatal day 21. The animals were sacrificed immediately after undergoing electroretinogram (ERG) and optomotor response (OMR) experiments 2 weeks later. In Group 2, one eye of RCS rats received sequential intravitreal injections with the same material on postnatal days 21 and 35. Visuomotor response and retinal conductance experiments were performed 2 weeks after the injection schedule was completed, and animals were sacrificed for further analysis.

### Experimental Example 10. Electroretinography

Scotopic ERG was used to evaluate functional changes in rat retinal photoreceptors. Before ERG recordings, animals were kept in a dark room for 16 hours. All recording procedures were performed under dim red light (λ > 600 nm). Hydroxypropyl methylcellulose gel was applied to the cornea and covered with gold-ring contact electrodes, and the reference and ground electrodes were placed subcutaneously in the tail and ear regions, respectively. In order to reduce the signal-to-noise ratio, responses were obtained by averaging 3 to 5 signals at 15-second intervals. The signal was amplified and filtered through a digital band-pass filter from 3 Hz to 500 Hz to generate a- and b-waves. White flash stimulation was delivered using a Ganzfeld stimulator (UTAS-3000; LKC Technologies, Gaithersburg, MD, USA). For scotopic ERG recordings, the eyes of rats adapted to darkness for at least 16 hours were exposed to flash intensities ranging from -2 to 1.5 cd.s/m². In the dark adaptation response, the amplitude of the a-waves was measured from the baseline to the negative peak, and the amplitude of the b-waves was measured from the lowest point of the a-wave to the next response peak. All animals (n = 6/group) were recorded for baseline recording before starting experimental procedures. The next day after the experiment, all animals were recorded again, regardless of whether the 660 nm light treatment paradigm was used.

### Experimental Example 11. Optomotor reaction experiment

OMR was measured with the OptoMotry system (Cerebral-Mechanics, Lethbridge, Canada). RCS rats were acclimatized to ambient lighting for 30 minutes and then placed on a stimulation platform surrounded by four computer monitors displaying black and white vertical striped patterns. Visual detection was considered successful when the RCS rat stopped moving and reflexively turned its head to begin tracking the movement of the stripes. Afterwards, the detection threshold was obtained from the optomotry software.

### Experimental Example 12. Primary rat RPE cell culture

Primary cultures of rat RPE cells were established in Sprague-Dawley rats. After removing the cornea, lens and retina, the posterior eye cup was immersed in fresh Hank's balanced salt solution (Thermo Fisher Scientific). Next, posterior eyecups were then incubated with dispase (StemCell Technologies) for 30 minutes at 37°C. Then, RPE cells were separated from the basement membrane (Bruch's) and collected through gentle aspiration. The harvested RPE cells were plated on 35-mm Matrigel-coated dishes (Corning Life Sciences) including the 'Miller' medium (DMEM supplemented with 20% FBS, N1 medium supplement, 2 mM GlutaMAX, 250 mg/mL taurine, 10 ng/mL hydrocortisone, 13 ng/mL triiodothyronine, and 1% penicillin/streptomycin). When the cells reached a confluent level, they were subcultured. For the H₂O₂ treatment group, cells were exposed to 200 µM H₂O₂ (Sigma-Aldrich) for 2 hours. Afterwards, the cells were washed once with PBS, and then 50 µL of retinal organoid-derived exosomes labeled with a green fluorescent dye were added to the culture medium and further cultured for 24 hours.

### Experimental Example 13. Immunohistochemistry

RCS rats were sacrificed under deep anesthesia, the carotid artery was excised with 4% paraformaldehyde in 0.1 M PBS, and the eye cup was removed. After removal of connective tissue, cornea, iris and lens, the posterior eye cup was processed for further morphological studies. The posterior eye cup was fixed in 4% paraformaldehyde for 1 hour at 4°C and dehydrated in 30% sucrose solution for 12 to 16 hours at 4°C. The posterior eye cup was embedded in OCT compound and stored at -80°C. The eye cup was cut into 10 µm thick cross-sections and mounted on a fine glass slide (Matsunami Glass Inc., Ltd., Osaka, Japan). All retinal cuts were performed in a direction encompassing the 12:00 to 6:00 o'clock axis at the anatomical location of the eye and included both the upper and lower retina. Slides including the optic nerve head were dried at 50°C for 30 min and washed with PBS and Tween-20 (PBST) for 15 minutes. Slides were blocked for 1 hour with 5% donkey serum (Jackson ImmunoResearch, West Grove, PA, USA) in PBST, and cultured incubated at 4°C with 5% normal porcine serum for anti-rhodopsin 1:1,000 (Millipore, Burlington, MA), anti-recoverin 1:1,000 (Millipore), anti-M/L opsin 1:1,000 (Millipore), anti-S-opsin 1:1,000 (Millipore), p-p38 1:1,000 (Cell Signaling Technology), phosphorylated c-Jun N-terminal kinase (p-JNK) 1:1,000 (Cell Signaling Technology), and phosphorylated extracellular signal-regulated kinase 1/2 (p-ERK1/2) 1:1,000 (Cell Signaling Technology). After washing with PBST, 5% the slides were incubated for 2 hours at room temperature in 5% donkey serum with secondary Alexa Fluor 568 donkey anti-rabbit IgG 1:1,000 (Invitrogen) and Alexa Fluor 488 donkey anti-mouse IgG 1:1,000 (Invitrogen). After washing with PBST, Hoechst 33342 was incubated in PBS (Invitrogen) at room temperature for 1 minute. Tissue pieces were then washed, covered with coverslips using mounting medium (Dako, Santa Clara, CA, USA), and images were photographed with a fluorescence microscope (Leica DMi8; Leica).

### Experimental Example 14. TUNEL staining

Tissues were stained according to the protocol provided by the manufacturer (12156792910, In Situ Cell Death Detection Kit, TMR red, Roche Diagnostics). Samples were washed three times with PBST and detected with secondary antibody (Alexa Fluor 488, dilution 1:1000; Invitrogen Corp., Carlsbad, CA). Nuclear counterstaining was performed with Hoechst 33,342 (Invitrogen Corp, Carlsbad, CA).

### Experimental Example 15. Real-time quantitative polymerase chain reaction (qRT-PCR)

Total RNA from RCS rat retina was isolated using TRIzol^{®} reagent (Invitrogen, Rockville, MD), and primary cDNA was synthesized using the SuperScript III First-Strand Synthesis System (Invitrogen) according to the manufacturer's protocol. cDNA was subjected to PCR using the StepOnePlus Real-Time PCR System (Applied Biosystems; Thermo Fisher Scientific, Inc.) using the QuantiSpeed SYBR Hi-ROX kit (Philekorea, Korea). PCR conditions were as follows; Polymerase activation at 95°C for 2 minutes, repeated 40 times for 5 seconds at 95°C (denaturation) and 30 seconds at 60°C (annealing/extension). Melt analysis was performed at each step: 95° for 15 seconds, 60°C for 1 minute and 95°C for 15 seconds. GAPDH (Glyceraldehyde 3-phosphate dehydrogenase) was used as an internal standard molecule, and mRNA expression levels were quantified using the 2-ΔΔCT method. The primer sequences used can be found in Table 1.

**[Table 1]**

| Gene | Forward 5'-3' | Reverse5'-3' |
|---|---|---|
| MAPK2K4 | AGAGACTGAGAACCCACAGCAT (SEQ ID No: 1) | CTACTCCGCATCACTACATCCA (SEQ ID No: 2) |
| MAPK1 | TGTTGCAGATCCAGACCATG (SEQ ID No: 3) | CAGCCCACAGACCAAATATCA (SEQ ID No: 4) |
| MAPK8 | ATTTGGAGGAGCGAACTAAG (SEQ ID No: 5) | CTGCTGTCTGTATCCGAGGC (SEQ ID No: 6) |
| MAPK10 | TCGAGACCGTTTCAGTCCAT (SEQ ID No: 7) | CCACGGACCAAATATCCACT (SEQ ID No: 8) |
| c-FOS | GTCCGTCTCTAGTGCCAACTTTAT (SEQ ID No: 9) | GTCTTCACCACTCCCGCTCT (SEQ ID No: 10) |
| c-Jun | TCCCCCATCGACATGGAGTCTC (SEQ ID No: 11) | CCAGTCCATCTTGTGTACCCTTG (SEQ ID No: 12) |
| GAPDH | CTGAGTATGTCGTGGAGTCTA (SEQ ID No: 13) | CTGCTTCACCACCTTCTTGAT (SEQ ID No: 14) |
| MAPK2K4, mitogen-activated protein kinase kinase 4; MAPK, mitogen-activated protein kinase; GAPDH, glyceraldehyde 3-phosphate dehydrogenase. | | |

### Experimental Example 16. Western Blot Analysis

Cells were lysed in whole cell extraction buffer including 10mM HEPES (pH 7.9), 0.1mM EDTA, 0.1mM EGTA, 5% glycerol, 1mM DTT and 400mM NaCl. Protein concentration was measured using the BCA protein assay kit according to the manufacturer's instructions (Thermo Scientific, Rockford, IL). Protein samples from cell lysates were mixed with an equal volume of 5 X SDS sample buffer, boiled for 5 minutes, and separated on a 9% SDS-PAGE gel. After electrophoresis, proteins were blotted with a PVDF membrane. The PVDF membrane was blocked for nonspecific binding with 3% BSA for 1 hour and incubated with primary specific antibodies against pERK, ERK, pJNK, JNK, pp38 and p38 in PBS-T (0.1% Tween-20 in PBS) bands were visualized using chemiluminescence reagent (Atto, Tokyo, Japan) and azure imaging system (Azure Biosystems, Dublin, CA). The fluorescence intensity of the bands was analyzed using ImageJ 1.53 software (National Institutes of Health, Bethesda, MD).

### Experimental Example 17. Isolation and sequence analysis of miRNAs from retinal organoid-derived exosomes

Exosomal RNA was extracted using TRIzol reagent (Invitrogen, Carlsbad, CA, USA) according to the manufacturer's instructions. The quality of RNA was assessed with an Agilent 2100 bioanalyzer using an RNA 6000 Pico Chip (Agilent Technologies, Amstelveen, The Neterlands), and RNA was quantified using a NanoDrop 2000 spectrophotometer system (Thermo Fisher Scientific, Waltham, MA, USA). The miRNA library was constructed using the NEVNext Multiplex Small RNA Library Prep Kit (New England BioLabs, Inc. USA) according to the manufacturer's instructions.

In summary, for library construction, total RNA for each sample was bound to the adapter, and then, cDNA was synthesized by reverse transcription using adapter-specific primers. PCR was performed to amplify the library, and the library was purified using the QLAquick PCR purification kit (Quaigen, Inc, German) and PAGE gel. The yield and size distribution of the small RNA library were evaluated using the Agilent 2100 Bioanalyzer instrument (Agilent Technologies, Inc. USA) for high-sensitivity DNA testing. High-throughput sequences were generated by single-end 75 nucleotide sequencing on the NextSeq500 system (Illumina, SanDiego, CA. USA).

### Experimental Example 18. Total RNA isolation and sequence analysis of RCS rat retina treated with retinal organoid-derived exosomes

Total RNA from RCS rat retina treated with exosomes was extracted using Trizol reagent (Invitrogen, Carlsbad, CA, USA) according to the manufacturer's instructions. The quality of the isolated total RNA was assessed with an Agilent 2100 bioanalyzer using an RNA 6000 Pico Chip (Agilent Technologies, Am stelveen, The Nerlant Technologies), and RNA was quantified using a NanoDrop 2000 spectrophotometer system (Thermo Fisher Scientific, Waltham, MA, USA). The isolated total RNA was used to create a library using the NEBnext Ultra II Directional RNA-Seq Kit (NEW ENGLANG BioLabs, Inc., UK) according to the manufacturer's instructions. Briefly, mRNA was isolated from total RNA using the Poly(A) RNA Selection Kit (LEXGEN, Inc., Austria). The isolated mRNA was synthesized and sheared into cDNA according to the manufacturer's instructions. Indexing was done using Illumina index 1-12, and amplification was performed using PCR. Afterwards, the library was screened using TapeStation HSD1000 screen tape (Agilent Technologies, Amstelveen, The Netherlands) to estimate average fragment size. For quantification, a library quantification kit using the Step One Real-Time PCR system (Life Technologies, Inc., USA) was used. High throughput sequencing was performed using NovaSeq 6000 (Illumina, Inc., USA) with paired-end 100 sequencing.

### Experimental Example 19. Corneal epithelial debridement

Under deep sedation and local anesthesia using 5% proparacaine, a 2 mm epithelial removal was made in the central part of the cornea with a sterile disposable biopsy punch (Kai, Tokyo, Japan). After wounding, 50 µL of exosome suspension including 1.0 × 10⁶ exosomes/µL or saline was instilled once to the cornea as an untreated control immediately after removal of the corneal epithelium.

The wound healing process was observed at 12-hour intervals from 0 to 36 hours using fluorescein staining dye, and the epithelial wound area was photographed with a camera equipped with a biomicroscope. Using ImageJ software, percent wound healing (%) was measured relative to the initial epithelial nodular area.

### Experimental Example 20. Tissue preparation and EdU assay

In order to analyze epithelial cell proliferation, 100 mg/kg of EdU (5-ethyl-2'-deoxyuridine) was injected intraperitoneally to treatment group rats administered with exosomes after creating a wound on the corneal surface and control group non-treated rats administered with a vehicle (PBS). 24 hours after wounding and treatment, mice rats deeply anesthetized and injected intracardiacally with 0.1 M normal saline (PBS) including 150 U/mL of heparin, and 4% phosphate buffer (PB) in 0.1 M. PFA (paraformaldehyde) was injected.

Afterwards, the eye was extracted, and a 360-degree sclerotomy was performed just behind the limbus of the eye to separate the lens and the posterior segment of the eye from the anterior segment of the eye.

Tissues were fixed in 4% PFA, cultured overnight in a PBS solution including 30% sucrose, and placed in OCT compound (Optimal Cutting Temperature compound). Sections were cut at 10 mm intervals and mounted on adhesive microscope slides (Histobond; Marienfeld-Superior, Lauda-Kϕnigshofen, Germany).

Corneal sections were washed in 3% BSA in PBS for 2 minutes and then washed with saline solution for 2 minutes. The proliferative activity of cells was measured by EdU assay using the Click-iT EdU Imaging kit (Invitrogen).

Nuclei were visualized after staining with Hoechst 33342 dye solution diluted 1:2,000 in PBS for 2 minutes at room temperature. After washing, tissue sections were covered with a coverslip using mounting medium (Dako, Santa Clara, CA) and imaged with a confocal microscope (Leica DMi8, Wetzlar, Germany).

### Experimental Example 21. Real-time quantitative polymerase chain reaction (qRT-PCR; Quantitative reverse transcription-polymerase chain reaction)

cDNA was amplified on a StepOnePlus^{™} Real-Time PCT system (Applied Biosystems; Thermo Fisher Scientific, Inc.) using primers for each target gene and the QuantiSpeed SYBR Hi-Rox kit (PhieKorea, Seoul, Korea). Polymerase reaction (PCR) was performed by repeating a process of DNA denaturation for 5 seconds at 95°C and annealing/extension for 30 seconds at 60°C 40 times. At each step, melting curve analysis was performed at 95°C for 15 seconds, 60°C for 1 minute, and 95°C for 15 seconds to confirm the specificity of the polymerase reaction product. GAPDH was used as an internal standard molecule, and mRNA expression levels were quantified using the 2-ΔΔCT method.

### Experimental Example 22. Data Analysis

Exosomal miRNA sequence reads were mapped using the bowtie2 software tool to obtain a bam file (alignment file). In this case, the mature miRNA sequence was used as a reference sequence for mapping. Read counts mapped to mature miRNA sequences were extracted from the alignment files using Bioconductor using bedtools (v2.25.0) and the R (version 3.2.2, R development Core Team, 2011) statistical programming language. Read counts were used to determine the expression levels of miRNAs. Standardization between samples was performed using the quantile normalization method of EdgeR, which is an R program package. Target genes of miRNAs were derived using miRNET (www.mimet.ca), and genes that were common targets of two or more miRNAs were selected.

Raw sequencing data quality assessment was performed on sequence reads of RCS rat retinal mRNA using FastQC (https//www.bioinformatics.babraham.ac.uk/projects/fastqc/)). After removing adapters and low-quality reads (<Q20) using FASTX_Trimmer (http://hannonlab.cshl.edu/fastx_toolkit/) and BBMap (https://sourceforge.net/projects/bbmap/)), trimmed reads were mapped to the reference genome using HISAT2 (http://daehwankimlab.github.io/hisat2/main/), and read counts were extracted using HTseq. Data were processed according to the FPKM+Geometric normalization method, and differentially expressed gene (DEG) analysis was performed with the exactTest function of EdgeR, which is an R package (R development Core Team, 2020). The construction of miRNAs was performed using the NEBNext Multiplex Small RNA Library Prep kit (New England BioLabs) according to the manufacturer's instructions.

Gene ontology analysis and pathway analysis of the derived exosomal miRNA target genes and RCS rat retina mRNA genes were performed using the R package gprofiler (biit.cs.ut.ee/gprofiler) and the Cytoscape program (version 3.5, cytoscape.org) package ClueGO (version 2.5.8., apps.cytoscape.org/apps/cluego), and then visualized.

In order to perform Gene Set Enrichment Analysis (GSEA) to predict the function of abundant exosomal miRNAs related to inflammation, the gene sets including the terms "inflammation", "cell adhesion" or "epithelial cell" in the biological process of the Gene Ontology database (http://geneontology.org/) were transformed into gmt files including the predicted miRNA sets for each Gene Ontology (GO). The Gmt file was used for GSEA's preranked test for exosomal miRNAs, sorted in descending order of normalized cpm values.

### Experimental Example 23. Statistical Analysis

All experiments were independently repeated at least three times. The statistical significance of differences was analyzed by one-way analysis of variance (ANOVA), two-way analysis of variance, or paired t-test using GraphPad Prism 9 software (GraphPad Software, Inc., San Diego, CA, USA). Significant differences are indicated with a single (^{*},^{#}; p < 0.05), double (**,^{##}; p < 0.01), or triple asterisk (***,^{###}; p < 0.001).

### Example 1. Formation of retinal organoids

As can be confirmed in FIG. 1, aggregates derived from human induced pluripotent stem cells formed embryoid bodies and ultimately formed three-dimensional retinal organoids. After 28 days of differentiation, the retinal organoid periphery showed bright, stratified layers, which are unique morphological and phenotypic characteristics. Retinal organoids were fluorescently stained with markers such as CHX10, Ki67 and HuC/D expressed in early retinal organoids, and their expression was confirmed to be increased. It was confirmed that the 60-day organoid had the characteristics of early retinal formation consisting of retinal neuroblasts and retinal ganglion cells, which means that retinal organoids appropriate for the time were successfully formed.

### Example 2. Extracellular vesicles in retinal organoid culture

In order to determine whether 3D retinal organoids derived from human induced pluripotent stem cells release exosomes, organoid culture fluid was analyzed at 60 days. As can be confirmed in FIG. 2, extracellular vesicles (EVs) isolated from the culture medium of retinal organoids were analyzed by electron microscopy (TEM) and showed spherical and cup shapes that were consistent with the size and shape of known extracellular vesicles. As a result, it was confirmed that extracellular vesicles were successfully isolated from the 60-day organoid culture. Additionally, we measured the size and concentration of extracellular vesicles using nanoparticle tracking analysis (NTA). The diameter of extracellular vesicles ranged from 30 nm to 550 nm, with an average diameter of 149.5 ± 2.9 nm and an average peak diameter of 72 ± 1.8 nm. According to diameter analysis, extracellular vesicles produced from retinal organoids included both exosomes and microvesicles, and the majority of extracellular vesicles were exosomes with a diameter of 30 to 150 nm. The concentration of extracellular vesicles produced from retinal organoids was also analyzed, and the average concentration was 4.54 × 10¹⁰ ± 5.86 × 10⁸ particles/mL.

Using electron microscopy, retinal organoid-derived exosomes were characterized in more detail and found to have a cup shape with an average diameter of approximately 110 nm. As a result of immunoblotting analysis, it showed that the levels of exosome markers CD9, CD63 and CD81 in the isolated exosomes were higher than those in the crude precipitate of the RO culture supernatant. On the other hand, cell-specific markers β-actin and calnexin were not detected in isolated exosomes.

### Example 3. Effect of retinal organoid-derived exosomes on reducing apoptosis in photoreceptors of RCS rats

In order to determine whether exosomes have any effect on apoptosis in photoreceptor apoptosis in RCS rats, the experiments were conducted a group (Group 1) that received a single injection of exosomes obtained from 60-day intravitreal retinal organoid cultures of RCS rats at 3 weeks and evaluated the therapeutic effect at 5 weeks, and a group (Group 2) that received two injections at 3 and 5 weeks and evaluated the effect at 7 weeks, and the results are shown in FIG. 3.

The inventors of the present invention investigated the effect of retinal organoid-derived exosomes on photoreceptor apoptosis using TUNEL staining. Whereas many TUNEL-positive cells were detected in the outer nuclear layer (ONL) of untreated retinas and vehicle (PBS, phosphate buffer solution)-treated eyes, the number of TUNEL-positive cells in the treated eyes in the retinas of Groups 1 and 2 treated with retinal organoid-derived exosomes was significantly reduced (B and C of FIG. 3a, and E and F of FIG. 3b). The thickness of the outer nuclear layer was significantly preserved in eyes treated with retinal organoid-derived exosomes in Groups 1 and 2 compared to untreated and vehicle-treated eyes (D of FIG. 3a and G of FIG. 3b). Specifically, in Group 1, the outer nuclear layer thickness was preserved more in the central retina than in the peripheral retina, but in Group 2, the outer nuclear layer thickness was similarly preserved not only in the central retina but also in the peripheral retina.

### Example 4. Effect of retinal organoid-derived exosomes on improving visual function

In order to determine whether retinal organoid-derived exosomes improve visual function, scotopic ERG and OMR tests were performed on Groups 1 and 2 for 5 and 7 weeks, respectively. According to the morphological transformation of photoreceptor cells, the group treated with retinal organoid-derived exosomes had preserved both scotopic a- and b-waves compared to the control and vehicle (PBS)-treated groups. In addition, the inventors of the present invention also examined the visual acuity of RCS rats using the OMR test, which assesses motor responses under optical conditions and measures visual acuity by virtually rotating stripes of different widths. OMR also showed that treatment with retinal organoid-derived exosomes significantly preserved the vision of RCS rats compared to vehicle (PBS, phosphate buffer)-treated or untreated controls, which showed similar vision. These results demonstrated that retinal organoid-derived exosomes inhibit photoreceptor apoptosis and preserve visual function in RCS rats.

### Example 5. Protective effect of retinal organoid-derived exosomes on photoreceptors in RCS rats

In order to determine what kind of therapeutic effect retinal organoid-derived exosomes ultimately exhibit on photoreceptor cells in the degenerative retina in RCS rats, changes in photoreceptors were compared and analyzed through fluorescent staining of transverse retinal cross-sections with antibodies to photoreceptor markers recoverin, rhodopsin, M/L-opsin and S-opsin, and the results are shown in FIG. 4.

In both experimental groups, the fluorescence intensity of rhodopsin and recoverin in the retina treated with retinal organoid-derived exosomes was significantly higher than that in the control or vehicle (PBS, phosphate buffer)-treated retina. When the amounts of M/L-opsin and S-opsin were analyzed in the control, vehicle and retinal organoid-derived exosome-treated retinas, both experimental groups were significantly increased by treatment with retinal organoid-derived exosomes. This result means that exosomes have a protective effect on photoreceptors (A and C of FIG. 4a, and D and F of FIG. 4b).

### Example 6. Changes in gene expression profile of RCS rats by treatment with retinal organoid-derived exosomes

In order to determine the mechanisms underlying photoreceptor apoptosis, the inventors of the present invention used RNA-seq to profile total RNA from RCS rat eyes before and after treatment with retinal organoid-derived exosomes. Total RNA-seq of RCS rat eyes showed that exosome treatment significantly increased the expression of retinal cell markers, including photoreceptors and retinal pigment epithelial cells, compared to the control group (FIG. 5E). There were 189 genes whose expression was increased by exosome treatment compared to the control group (fold change > 1, FDR (False Discovery rate) q < 0.05). Gene ontology analysis confirmed that these genes are involved in endocrine development, response to retinoic acid and visual perception (FIGS. 5A and B), among biological processes, and by cell composition, genes acting in the extracellular matrix (38.46%) and photoreceptor outer segments (17.31%) were found to be abundant (FIG. 5C and D).

Our data showed that downregulated genes were involved in pathways that regulate cell proliferation, differentiation and survival. These results suggest that MAPK, PI3K-Akt and RAS signaling pathways are abnormally activated in the retinas of Royal College of Surgeons (RCS) rats in which photoreceptor cell apoptosis progresses rapidly in the untreated negative control group and the vehicle, that is, the phosphate-buffered solution-injected group, whereas in the retinas treated with retinal organoid-derived exosomes, the expression of several genes involved in these signaling pathways was significantly reduced. However, with these results alone, there is a limitation to confirming whether the downregulated genes in these three signaling pathways actually activate or inhibit each signaling pathway, and thus, in the next step, the inventors of the present invention performed a process of analyzing the miRNA components included in the retinal organoid-derived exosomes used in the treatment.

### Example 7. miRNA analysis of retinal organoid-derived exosomes

In order to determine the therapeutic mechanism of these retinal organoid-derived exosomes, the inventors of the present invention analyzed the miRNA content in the retinal organoid-derived exosomes using RNA-seq profiling. In retinal organoid-derived exosomes, 128 miRNAs, more than 100 per million (cpm), were detected. Among these, the 20 most abundant miRNAs with more than 1,000 cpm are shown in FIG. 7. In target gene prediction, 4,929 human genes were targets of at least three of the 20 most abundant miRNAs. The biological processes of 293 genes targeted by more than 10 miRNAs were shown to be abundant in ontology terms such as 'regulation of cellular amide metabolism', 'regulation of cell development' and 'response to transforming growth factor beta'. In the pathway enrichment analysis, genes were enriched in the 'cancer development pathway', 'MAPK signaling pathway' and 'stem cell pluripotency regulation signaling pathway', and among these, the MAPK signaling pathway was selected as the key pathway. In order to evaluate the role of target genes regulated by retinal organoid-derived exosomal miRNAs, the inventors of the present invention compared predicted target genes and DEGs in RCS rats treated with exosomes. Among the 4,929 human predicted target genes, 301 genes out of 4,674 rat homologs were significantly downregulated in vesicle-treated eyes compared with non-vesicle-treated eyes (p <0.05). These genes were mainly shown to be abundant in the MAPK signaling pathway (61.22%).

Genes enriched in the MAPK pathway were DUSP16, EGFR, FOS, MAP2K4, MAPK1, MAPK8, MAPT, NF1, RASA2, RASGRP1, RPS6KA5 and TEK in retinal organoid-derived exosomes. Among the 20 most abundant miRNAs in retinal organoid-derived exosomes, 15 were predicted to target these 12 genes. Among these, hsa-let-7b-5p can target 11 out of 12 genes, suggesting that hsa-let-7b-5p may be a key miRNA that regulates the MAPK pathway.

### Example 8. Identification of the inhibitory effect of retinal organoid-derived exosomes on the MAPK signaling pathway by miRNA

Total RNA sequence analysis of RCS rat retina confirmed that retinal organoid-derived exosome treatment significantly reduced MAPK pathway gene expression compared to the control group. In order to confirm these results, the inventors of the present invention performed qRT-PCR analysis and found that the expression of MAPK-related genes, including MAP2K4, MAPK1, MAPK8, MAPK10, c-FOS and c-JUN, was significantly lower in retinal organoid-derived exosome-treated retinas than in control retinas (FIG. 7B).

### Example 9. Confirmation of therapeutic effect of retinal organoid-derived exosomes in oxidative stress model of RPE cells

Following the *in vivo* experiments, in order to determine the effects of retinal organoid-derived exosomes using another *in vitro* model, the inventors of the present invention established a retinal pigment epithelial (RPE) cell model for oxidative stress damage by treating primary rat RPE cells with specific H₂O₂ concentrations. In order to investigate the uptake of exosomes, calcein-labeled retinal organoid-derived exosomes were incubated with cultured RPE cells for 30 minutes. Green fluorescein particles were observed throughout the cytoplasm (FIG. 10A). In the group treated with retinal organoid-derived exosomes, the expression of p-JNK/JNK and pERK/ERK was significantly suppressed compared to the group treated with H₂O₂ (FIGS. 10B and C). In addition, the inventors of the present invention investigated the anti-apoptotic effect of retinal organoid-derived exosomes. As expected, the expression level of cleaved caspase-3 was significantly suppressed in the group treated with retinal organoid-derived exosomes compared to the H₂O₂ treated group.

### Example 10. Effect of retinal organoid (RO)-derived exosomes on epithelial wound treatment

In order to measure the wound healing effect of retinal organoid-derived exosomes on the corneal epithelium, a 2 mm-sized damage was made in the rat corneal epithelium, retinal organoid-derived exosomes were injected into the cornea as an experimental group, and a vehicle (PBS, phosphate buffer solution) was injected into the cornea as a control group.

Through tissue photographs, the cornea treated with the vehicle (PBS, phosphate buffer solution) 36 hours after treatment showed blurring, which is commonly found in corneas, but a relatively clear corneal treatment effect could be observed in the cornea treated with retinal organoid-derived exosomes.

In addition, there was no significant effect on the treatment of corneal epithelial wounds in the first 12 hours, but at 24 to 36 hours, a significantly improved treatment effect was shown in the group treated with retinal organoid-derived exosomes compared to the control group treated with the vehicle. (**P < 0.001). Through this, it can be seen that the group treated with retinal organoid-derived exosomes showed a faster therapeutic effect than the control group treated with a vehicle (PBS, phosphate buffer solution).

### Example 11. Improved wound healing effect through cell proliferation of retinal organoid (RO)-derived exosomes

In order to investigate possible mechanisms in the improved wound healing process, EdU investigation was performed to compare the degree of cell proliferation in the experimental group where the cornea was treated with retinal organoid-derived exosomes and the control group where the cornea was treated with vehicle (PBS). As can be seen in FIG. 4A, the degree of proliferation of epithelial cells in the leading edge, periphery and limbus of the cornea was significantly increased in corneas treated with retinal organoid-derived exosomes compared to corneas treated with the vehicle. This means that retinal organoid-derived exosomes increase the degree of cell proliferation and improve the wound healing effect.

In addition, more EdU-positive cells were observed in corneas treated with retinal organoid-derived exosomes than in control corneas treated with vehicle (*p < 0.05, **p < 0.001.).

### Example 12. Improved wound healing effect through anti-inflammatory action of retinal organoid-derived exosomes

The inventors of the present invention investigated whether treatment with retinal organoid-derived exosomes had an anti-inflammatory effect, which is known to play an important role in the wound healing process. In order to evaluate the potential anti-inflammatory action of exosomal miRNAs, GSEA analysis was performed on 282 miRNAs detected at least 10 cpm in 21 GO (Gene Ontology) terms including "inflammatory", "cell adhesion" or "epithelial cell" (FDR q<0.05). Abundant miRNAs were significantly expressed in "inflammatory cell apoptotic process", "cytokine production involved in inflammatory response", "neuroinflammatory response", "inflammatory response to wounding" and "regulation of chronic inflammatory response" (FDR q<0.001).

miRNAs targeting genes in these biological processes were mainly abundant in the top 20. In particular, exosomal miRNAs targeting TNF, CCL2, CCL5 or IL-6 were present in the top 10. These results indicate that abundant exosomal miRNAs derived from retinal organoids have the effect of preventing inflammatory responses by inducing corneal healing.

By using qRT-PCR, the expression levels of inflammatory cytokines TNF-α, IL-6, CCL2 and CCL5 were quantitatively compared between the group treated with retinal organoid-derived exosomes to the cornea and the group treated with the vehicle (PBS) (** p < 0.001). Compared to corneas treated with the vehicle, the levels of all four types of inflammatory cytokines were reduced in corneas treated with retinal organoid-derived exosomes. This indicates that the anti-inflammatory effect of retinal organoid-derived exosomes plays an important role in wound healing of the corneal epithelium.

The present invention is an invention that has been performed through the following project.
[National research and development project that supported this invention]
[Project Identification Number] 1465035564
[Project Number] HI21C0317010022
[[Name of Ministry] Ministry of Health and Welfare
[Name of Project Management (Specialized) Institution] Korea Health Industry Development Institute
[Title of Research Project] Disease-centered translational research project
[Title of Research Task] Establishment of organoids for the treatment of retinal diseases and identification of treatment effects
[Name of Task Performance Institution] Soonchunhyang University Industry-Academic Cooperation Foundation
[Research Period] January 1, 2022 to December 31, 2022

## Claims

1. A pharmaceutical composition for preventing or treating an eye disease, comprising exosomes derived from retinal organoids.

2. The pharmaceutical composition of claim 1, wherein the exosomes comprise at least one miRNA selected from the group consisting of hsa-miR-122-5p, hsa-miR-3591-3p, hsa-miR-99a-5p, hsa-miR-148a-3p, hsa-let-7a-5p, hsa-let-7b-5p, hsa-let-7c-5p, hsa-let-7f-5p, hsa-miR-125a-5p, hsa-miR-100-5p, hsa-miR-4443, hsa-miR-26a-5p, hsa-miR-128-3p, hsa-miR-27b-3p, hsa-miR-126-3p, hsa-miR-10a-5p, hsa-miR-99b-5p, hsa-let-7e-5p, hsa-miR-21-5p and hsa-miR-30d-5p.

3. The pharmaceutical composition of claim 1, wherein the eye disease is a degenerative retinal disease.

4. The pharmaceutical composition of claim 3, wherein the degenerative retinal disease includes a hereditary retinal disease, an acquired degenerative retinal disease, retinopathy of prematurity and optic neuropathy.

5. The pharmaceutical composition of claim 4, wherein the hereditary retinal disease includes retinitis pigmentosa, X-linked juvenile retinoschisis, Leber congenital amaurosis, Stargardt disease, choroideremia and gyrate-atrophy, and the acquired degenerative retinal disease includes wet age-related macular degeneration, dry age-related macular degeneration, diabetic retinopathy, hypertensive retinopathy, retinal detachment, macular hole, macular telangiectasia (MacTel), retinal degeneration, macular degeneration, retinal vein occlusion, retinal artery occlusion and glaucoma.

6. The pharmaceutical composition of claim 1, wherein the eye disease includes an ocular surface disease of corneal epithelial tissue, stroma and endothelial tissue.

7. The pharmaceutical composition of claim 6, wherein the ocular surface disease includes genetic damage, inflammatory damage, immunological damage, traumatic damage, dry eye, conjunctival lesions, conjunctivitis, keratoconjunctivitis, corneal epithelial tissue damage, corneal stromal damage, corneal endothelial tissue damage, dry eye syndrome and eye strain.

8. The pharmaceutical composition of claim 7, wherein the corneal epithelial tissue damage includes corneal abrasion, keratitis and corneal dystrophy, and the corneal stromal damage and corneal endothelial tissue damage include corneal laceration due to trauma, opacity, inflammatory keratitis, non-inflammatory keratitis and corneal dystrophy.

9. The pharmaceutical composition according to any one of claims 6 to 8, wherein the pharmaceutical composition has effects of improving proliferation of corneal epithelial cells, corneal edema, opacity, immunosuppression and antiinflammatory effects.

10. The pharmaceutical composition according to any one of claims 1 to 9, wherein the pharmaceutical composition is for intravitreal injection, subretinal injection and topical ocular administration in the form of eye drops.

11. The pharmaceutical composition according to any one of claims 1 to 10, wherein the pharmaceutical composition is any one formulation selected from the group consisting of a skin external preparation, an injection, an infusion, a spray, a liquid, an ointment, a suspension, a syrup, an emulsion, a powder, a granule, a tablet, a sustained-release preparation, an eye drop, a capsule, an intraocular implant, an intraocular injection, a contact lens cleaner and a contact lens lubricant.

12. A pharmaceutical preparation for preventing or treating an eye disease, comprising the pharmaceutical composition according to any one of claims 1 and 2, wherein the eye disease includes a degenerative retinal disease and an ocular surface disease of corneal epithelial tissue, stroma and endothelial tissue.

13. The pharmaceutical preparation of claim 12, wherein the degenerative retinal disease includes a hereditary retinal disease, an acquired degenerative retinal disease, retinopathy of prematurity and optic neuropathy.

14. The pharmaceutical preparation of claim 13, wherein the hereditary retinal disease includes retinitis pigmentosa, X-linked juvenile retinoschisis, Leber congenital amaurosis, Stargardt disease, choroideremia and gyrate-atrophy, and the acquired degenerative retinal disease includes wet age-related macular degeneration, dry age-related macular degeneration, diabetic retinopathy, hypertensive retinopathy, retinal detachment, macular hole, macular telangiectasia (MacTel), retinal degeneration, macular degeneration, retinal vein occlusion, retinal artery occlusion and glaucoma.

15. The pharmaceutical preparation of claim 12, wherein the ocular surface disease includes genetic damage, inflammatory damage, immunological damage, traumatic damage, dry eye, conjunctival lesions, conjunctivitis, keratoconjunctivitis, corneal epithelial tissue damage, corneal stromal damage, corneal endothelial tissue damage, dry eye syndrome and eye strain.

16. The pharmaceutical preparation of claim 15, wherein the corneal epithelial tissue damage includes corneal abrasion, keratitis and corneal dystrophy, and the corneal stromal damage and corneal endothelial tissue damage include corneal laceration due to trauma, opacity, inflammatory keratitis, non-inflammatory keratitis and corneal dystrophy.

17. A food composition for preventing or ameliorating an eye disease, comprising exosomes derived from retinal organoids, wherein the eye disease includes a degenerative retinal disease and an ocular surface disease of corneal epithelial tissue, stroma and endothelial tissue.
